# EUROPÄISCHE PATENTANMELDUNG

(11) **EP 0 701 795 A1**
(43) Veröffentlichungstag der Anmeldung: **20.03.1996**
(21) Anmeldenummer: 95202441.2
(22) Anmeldetag: 08.09.1995
(51) Int. Cl.: A61B 6/00

(54) **Röntgenuntersuchungsgerät**

(30) Priorität: 16.09.1994 DE 4433036
(71) Anmelder: Philips Patentverwaltung GmbH, D-22335 Hamburg (DE); Philips Electronics N.V., NL-5621 BA Eindhoven (NL)
(72) Erfinder: Linhart, Claus, Dr.-Ing., Röntgenstrasse 24, D-22335 Hamburg (DE)
(74) Vertreter: Hartmann, Heinrich, Dipl.-Ing.

(57) **Zusammenfassung**

Die Erfindung betrifft ein Röntgenuntersuchungsgerät (1) mit einem Patientenlagerungstisch (2) und einem relativ zum Patientenlagerungstisch (2) in mindestens zwei Raumrichtungen hin- und herbeweglichen Röntgenzielgerät (4), sowie in einen Bedienungsgriff (10) des Röntgenzielgerätes (4) integrierten Sensoren (8a,8b,8c,8d) zum Ansteuern mindestens eines Servomotors (5,7) für das Röntgenzielgerät. Um bei einer Verschiebbarkeit des Röntgenzielgerätes in zwei unterschiedlichen Raumrichtungen mit einem einzigen Bedienungsgriff (10) auszukommen, sieht die Erfindung vor, daß die Sensoren so angeordnet sind, daß bei Beaufschlagung des Bedienungsgriffs mit einer Kraft in einer beliebigen der mindestens vier möglichen Bewegungsrichtungen jeweils eine andere Gruppierung (8a,8b; 8c,8d; 8a,8c; 8b,8d) von Sensoren (8a,8b,8c,8d) aktiviert wird.

## Beschreibung

Die Erfindung betrifft ein Röntgenuntersuchungsgerät mit einem Patientenlagerungstisch und einem relativ zum Patientenlagerungstisch in mindestens einer Raumrichtung hin- und herbeweglichen Röntgenzielgerät, sowie durch einen Bedienungsgriff des Röntgenzielgerätes aktivierbaren Sensoren zum Ansteuern mindestens eines Servomotors des Röntgenzielgerätes.

Bei Röntgenuntersuchungsgeräten, insbesondere bei nahbedienten Röntgendurchleuchtungsgeräten wird das Röntgenzielgerät vom untersuchenden Arzt über einen auf dem Patientenlagerungstisch stationär gelagerten Patienten hinwegbewegt, um Anomalien schnell und damit ohne große Strahlenbelastung für den Patienten aufzufinden. Da das Röntgenzielgerät unter anderem einen Röntgenstrahler, einen Bildempfänger und einen Bildverstärker umfaßt und somit eine verhältnismäßig große Trägheitsmasse besitzt, werden bereits seit langem Servomotoren eingesetzt, um die vom Arzt in den Bedienungsgriff des Röntgenzielgerätes eingeleiteten Kräfte so zu verstärken, daß sich das Röntgenzielgerät mühelos und ohne großen Kraftaufwand verschieben läßt. Die Verschiebung des Röntgenzielgerätes erfolgt dabei im allgemeinen in Längsrichtung des Patientenlagerungstisches und senkrecht dazu in Kompressionsrichtung auf den Patienten zu und von diesem weg.

Die Servomotoren sollen möglichst unauffällig arbeiten, d.h. die Kraftunterstützung soll möglichst ruckfrei und entsprechend der Stärke der vom Arzt in das Röntgenzielgerät eingeleiteten Kräfte in der gewünschten Raumrichtung erfolgen. Dazu dienen die durch den Bedienungsgriff aktivierten Sensoren, welche die vom Arzt auf eine Griffoberfläche des Bedienungsgriffs ausgeübte Kraft messen und dafür sorgen, daß vom Servomotor eine Unterstützungskraft mit der gewünschten Stärke bereitgestellt wird. Um die Bedienung zu erleichtern, sollte die Verstärkung im wesentlichen proportional zu der in der jeweiligen Raumrichtung eingeleiteten Kraft erfolgen. Eine derartige Verstärkung paßt zu natürlichen Bewegungsmustern wie beispielsweise einem großen Krafteinsatz zum schnellen Anfahren eines gewünschten Körperbereichs bzw. einem kleinen Krafteinsatz zum genauen Justieren des Zielgerätes und vermittelt damit dem Arzt subjektiv das Gefühl, daß ein Gerät mit sehr geringer Masse den von ihm ausgeübten Kräften folgt.

Aus der DE-OS-27 39 934 ist bereits ein Röntgenuntersuchungsgerät der eingangs genannten Art bekannt, bei dem der Bedienungsgriff eine in seiner Längsrichtung gegen zwei Anschlagflächen hin- und herverschiebbare Hülse umfaßt, wobei als Sensor jeweils eine zwischen einer Stirnfläche der Hülse und einer der Anschlagflächen angeordnete Scheibe aus druckabhängigem Widerstandsmaterial angeordnet ist, die Teil einer dem Steuerkreis des Servomotors zugeordneten Brückenschaltung ist. Bei Belastung der Scheibe mit einer Druckkraft wird die Brückenschaltung dekompensiert und die dabei in ihrer Diagonale auftretende Spannung mittels eines Operationsverstärkers verstärkt und an einen Differenzverstärker weitergeleitet, dessen Ausgangsspannung abgegriffen wird, um den Servomotor über einen Thyristorsatz anzutreiben. Bei dem bekannten Röntgenzielgerät lassen sich allerdings nur die in Längsrichtung des Bedienungsgriffs in diesen eingeleiteten Kräfte verstärken, so daß bei Röntgenzielgeräten mit zwei unterschiedlichen Bewegungsrichtungen zwei derartige Bedienungsgriffe erforderlich sind, was eine erheblich umständlichere Handhabung zu Folge hat.

Ausgehend hiervon liegt der Erfindung die Aufgabe zugrunde, ein Röntgenuntersuchungsgerät der eingangs genannten Art dahingehend zu verbessern, daß es mit einem einzigen Bedienungsgriff in zwei unterschiedlichen Raumrichtungen kraftverstärkt hin- und herbeweglich ist.

Diese Aufgabe wird erfindungsgemäß dadurch gelöst, daß das Röntgenzielgerät mit dem Bedienungsgriff in mindestens zwei unterschiedlichen Raumrichtungen verschiebbar ist, und daß die Sensoren in Bezug auf den Bedienungsgriff so angeordnet sind, daß bei Beaufschlagung des Bedienungsgriffs mit einer Kraft in einer beliebigen der mindestens vier möglichen Bewegungsrichtungen jeweils eine andere Gruppierung aus jeweils mindestens zwei der Sensoren aktiviert wird.

Eine weitere Ausgestaltung der Erfindung sieht vor, daß das Röntgenzielgerät in einer dritten Raumrichtung hin- und herverschiebbar ist, die vorzugsweise senkrecht zu den besagten, gleichfalls zueinander senkrechten Raumrichtungen ist, wobei die Sensoren so angeordnet sind, daß beim Einleiten einer Kraft mit einer Kraftrichtung, die mit einer beliebigen der sechs möglichen Bewegungsrichtungen übereinstimmt, jeweils eine bestimmte Gruppierung aus mindestens zwei Sensoren aktiviert wird, die sich jeweils von den Gruppierungen unterscheidet, welche aktiviert werden, wenn die Kraftrichtung eine andere ist und mit einer der fünf anderen möglichen Bewegungsrichtungen übereinstimmt. Alternativ oder zusätzlich kann eine Dreh- oder Schwenkbewegung des Röntgenzielgerätes um eine Schwenkachse vorgesehen sein, wobei die Sensoren ebenfalls so angeordnet sind, daß in Abhängigkeit von der Richtung einer eingeleiteten Kraft unterschiedliche Gruppierungen von Sensoren aktiviert werden. Beim Einleiten einer Kraft mit einem Kraftvektor, der aus zwei oder mehr der Raum- oder Drehrichtungen kombiniert ist, werden entsprechend mehrere vorzugsweise als Sensorpaare ausgebildete Sensorgruppierungen aktiviert, deren Signale dann jeweils zur Ansteuerung eines in der entsprechenden Bewegungsrichtung wirkenden Servomotors dienen.

Eine bevorzugte Ausgestaltung der Erfindung ist gekennzeichnet durch mindestens einen zwischen dem Bedienungsgriff und jedem Sensor angeordneten, verformbaren Kraftübertragungskörper aus einem inkompressiblen Material mit vorzugsweise elastischen Eigenschaften. Das Material zwischen dem Sensor und dem Bedienungsgriff dient als Druckübertragungsmedium, um eine von einer Bedienungsperson auf die Griffoberfläche ausgeübte Kraft in einen allseitig wirkenden Druck in einem oder mehreren der Kraftübertragungskörper umzuwandeln, wobei dieser Druck dann auf eine aktive Fläche des zweckmäßig als Druck- oder Kraftsensor mit hohem Signalhub und geringem Raumbedarf und bevorzugt als Foliendrucksensor ausgebildeten Sensors einwirkt und dort eine zum jeweiligen Druck proportionale Kraft erzeugt, die wiederum im Sensor eine entsprechende Widerstandsänderung bewirkt. Das inkompressible Material besitzt dabei vorzugsweise gummielastische Eigenschaften, so daß es beim Loslassen des Bedienungsgriffs in seine ursprüngliche Lage zurückkehrt. Eine vorzugsweise starre Verbindung zwischen dem Kraftübertragungskörper und dem Sensor kann beispielsweise durch Aufkleben des Sensors auf den Kraftübertragungskörper oder zweckmäßig durch Einbetten des mit dem Tragelement des Bedienungsgriffs verbundenen Sensors im Kraftübertragungskörper hergestellt werden. Obwohl die Sensoren und die Kraftübertragungskörper auch außerhalb des Bedienungsgriffs angeordnet sein können, sind sie vorzugsweise in diesen Griff integriert.

Der Bedienungsgriff kann so gestaltet sein, daß in die Kraftübertragungskörper nur Kräfte einleitbar sind, deren Kraftrichtungen parallel zu dem möglichen Bewegungsrichtungen sind, während Kräfte in anderen Raumrichtungen beispielsweise unmittelbar vom Röntgenzielgerät aufgenommen werden.

Eine weitere bevorzugte Ausgestaltung der Erfindung sieht vor, daß die einzelnen Sensor-Signale einer Auswerteschaltung zugeführt werden, die den Servomotoren nur dann Antriebsenergie zuführt, wenn alle Sensorsignale jeweils einer Gruppierung zumindest annähernd vergleichbar groß sind. Auf diese Weise kann das Röntgenuntersuchungsgerät bei Ausfall eines Sensors abgeschaltet und damit eine höhere Sicherheit gewährleistet werden.

Die Größe der Sensorsignale wird von der Größe der Kraftangriffsflächen bestimmt, über die der Bedienungsgriff auf die Kraftübertragungskörper einwirkt. Eine große Kraftangriffsfläche in einer Bewegungsrichtung, in der das Röntgenzielgerät eine verhältnismäßig kleine Trägheitsmasse aufweist, und eine kleine Kraftangriffsfläche in einer Bewegungsrichtung, in der das Röntgenzielgerät eine verhältnismäßig große Trägheitsmasse aufweist, hat bei Einleitung einer jeweils gleichgroßen Kraft unterschiedlich große Drücke im Kraftübertragungskörper zur Folge und damit bei gleicher Kennlinie der Kraftsensoren eine unterschiedlich große Kraftverstärkung durch die Servomotoren, was dazu führt, daß der Arzt beim Verschieben des Röntgenzielgerätes die unterschiedlich großen Trägheitsmassen nicht fühlbar wahrnimmt. Da die Trägheitsmassen bereits bei der Auslegung des Röntgenuntersuchungsgeräts bekannt sind, können sie bei der Dimensionierung der Kraftangriffsflächen berücksichtigt werden, so daß ggf. ein nachgeschalteter Verstärker entbehrlich wird.

Auf die beschriebene Weise läßt sich auch eine unterschiedlich große Kraftverstärkung erzielen, wenn das Röntgenzielgerät beispielweise bei gleicher Stärke der eingeleiteten Kraft in einer Raumrichtung z.B. in Längsrichtung des Patientenlagerungstisches stärker beschleunigt werden soll, als in einer anderen Raumrichtung, z.B. in Kompressionsrichtung.

Im folgenden wird die Erfindung anhand eines in der Zeichnung in schematischer Weise dargestellten Ausführungsbeispiels näher erläutert. Es zeigen:
- Fig. 1:: eine Seitenansicht eines erfindungsgemäßen Röntgenuntersuchungsgerätes;
- Fig. 2:: eine Längsschnitt durch den Bedienungsgriff des Röntgenuntersuchungsgeräts aus Fig. 1;
- Fig. 3:: einen Querschnitt durch den Bedienungsgriff;
- Fig. 4:: einen Querschnitt durch eine alternative Ausführungsform des Bedienungsgriffs;
- Fig. 5:: zwei perspektivische Querschnittsansichten durch eine weitere alternative Ausführungsform des Bedienungsgriffs;
- Fig. 6:: eine auseinandergeklappte Darstellung eines für einen Bedienungsgriff eines Röntgenuntersuchungsgerätes vorgesehenen Foliendrucksensors und
- Fig. 7:: eine Belastungs-/Widerstandskennlinie eines für einen Bedienungsgriff eines Röntgenuntersuchungsgerätes vorgesehenen Foliendrucksensors.

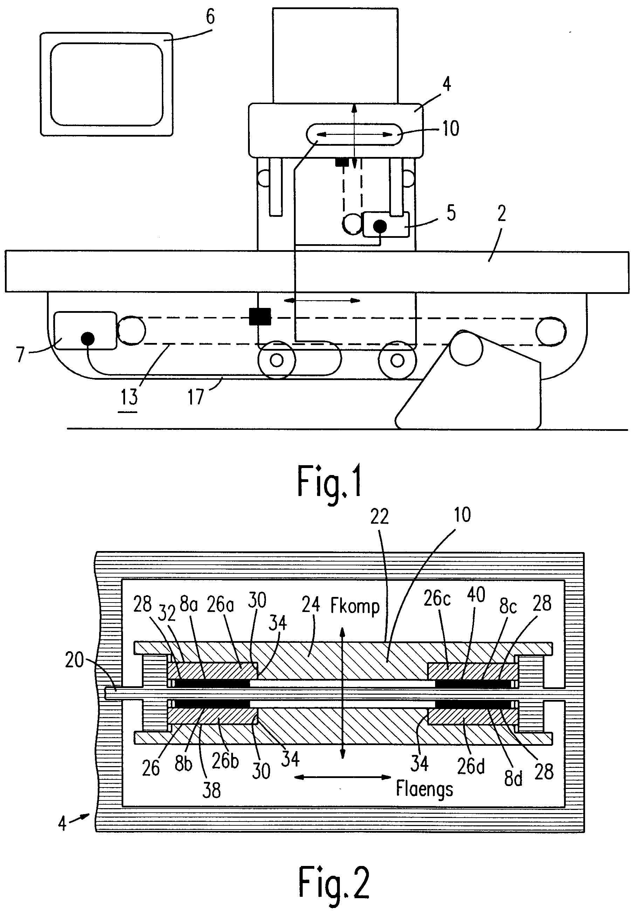

Das in Figur 1 schematisch dargestellte Röntgenuntersuchungsgerät 1 umfaßt im wesentlichen einen motorisch aus einer horizontalen Lage in eine vertikale Lage verschwenkbaren Patientenlagerungstisch 2, sowie ein in Längsrichtung des Patientenlagerungstisches 2 und in Kompressionsrichtung (senkrecht zum Patientenlagerungstisch 2) verfahrbares Röntgenzielgerät 4. Das Röntgenzielgerät 4 enthält u.a. einen Bildempfänger sowie einen Bildverstärker zur Umsetzung und Wiedergabe des vom Bildempfänger empfangenen Röntgenbildes auf einem Monitor 6, und ist mit einem unter dem Patientenlagerungstisch verfahrbaren Röntgenstrahler gekoppelt; es weist daher eine verhältnismäßig große Trägheitsmasse auf. Um das Hin- und Herverschieben des Röntgenzielgerätes 4 in Längsrichtung des Patientenlagerungstisches 2 und in Kompressionsrichtung zu erleichtern, sind Servomotoren 5,7 vorgesehen, die einen überwiegenden Teil der zum Bewegen des Röntgenzielgerätes 4 erforderlichen Kraft beisteuern, so daß es sich vom Arzt mühelos verschieben läßt (Fig. 1).

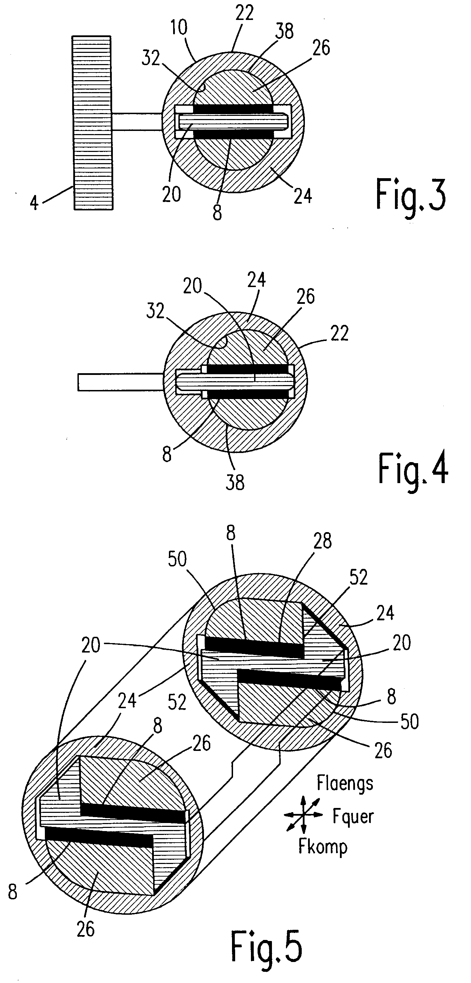

Die bedarfsgerechte Ansteuerung der Servomotoren 5,7 erfolgt mit Hilfe von vier als Foliendrucksensor ausgebildeten Sensoren 8a,8b,8c,8d, die in einem Bedienungsgriff 10 des Röntgenzielgerätes 4 integriert und über Steuerleitungen 17 mit den Servomotoren 5,7 verbunden sind (vergl. Fig. 2 und 3). Sie dienen dazu, eine vom Arzt in einer beliebigen der vier möglichen Bewegungsrichtungen des Röntgenzielgerätes 4 (d.h. jeweils in entgegengesetzten Richtungen in Längsrichtung des Patientenlagerungstisches 2 bzw. in Kompressionsrichtung) in den Bedienungsgriff 10 eingeleitete Kraft in Form einer Widerstandsänderung in jeweils zwei 8a,8b; 8c,8d; 8a,8c; 8b,8d der vier Foliendrucksensoren 8a,8b,8c,8d zu erfassen. Wie unten näher erläutert wird, nimmt der Widerstand der eingesetzten Foliendrucksensoren 8a,8b,8c,8d im wesentlichen proportional zu einem auf ihre aktive Fläche einwirkenden Druck in einem solchen Maß ab, daß die Widerstandsänderung mittels der Foliendrucksensoren 8a,8b,8c,8d unmittelbar und ohne weitere Verstärkung als Regelgröße für die Servomotoren 5,7 eingesetzt werden kann.

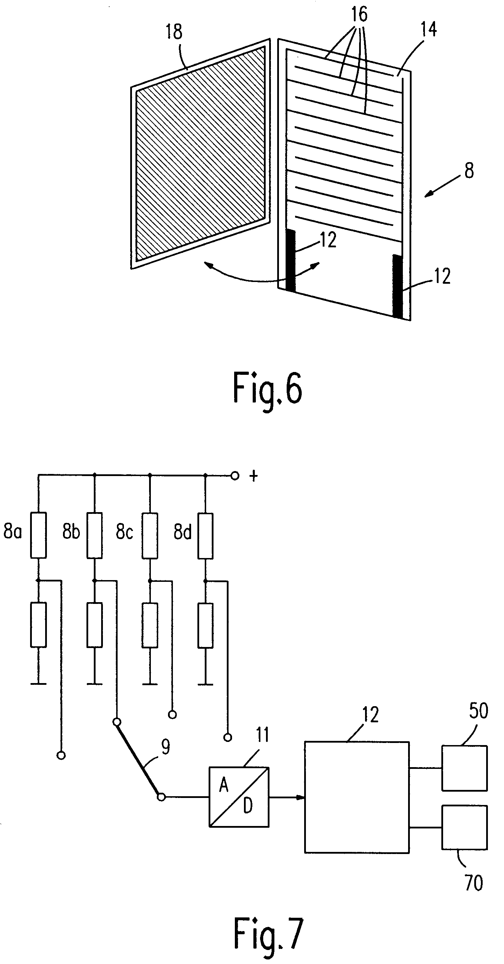

Die Foliendrucksensoren 8a,8b,8c,8d bestehen im wesentlichen aus zwei kammartig ausgebildeten Elektroden 12, die so auf einem Polymerfilm 14 aufgetragen sind (Fig. 6), daß ihre zueinander parallelen Leiterbahnelemente 16 ineinandergreifen, wobei der Abstand und die Breite der ineinandergreifenden Leiterbahnelemente 16 etwa 0,4 mm beträgt. Auf die vom Polymerfilm 14 abgewandte Oberseite der Elektroden 12 ist ein weiterer Film 18 aus einem Halbleiterpolymer aufgetragen (in Figur 6 aufgeklappt dargestellt), der die Elektroden 12 und den Polymerfilm 14 überdeckt, so daß die Leiterbahnelemente 16 durch den als Halbleiter ausgebildeten Film 18 parallelgeschaltet werden. Wird ein derartiger Foliendrucksensor 8a,8b,8c,8d auf einer seiner Breitseitenflächen gleichmäßig mit Druck beaufschlagt, so nimmt sein Widerstand im wesentlichen proportional zur Höhe des ausgeübten Drucks ab, wobei die Größenordnung der Widerstandsabnahme im wesentlichen derjenigen der Druckzunahme entspricht.

Der Bedienungsgriff 10 besteht im wesentlichen aus einem starr mit dem Röntgenzielgerät 4 verbundenen Tragelement 20, einem mit einer Griffoberfläche 22 versehenen Griffteil 24, den vier Foliendrucksensoren 8a,8b,8c,8d, sowie vier verformbaren Kraftübertragungskörpern 26, die jeweils zwischen einem Foliendrucksensor 8a,8b,8c,8d und dem Griffteil 24 angeordnet sind und aus einem inkompressiblen Material mit gummielastischen Eigenschaften bestehen. Die vier Foliendrucksensoren 8 sind mit zur Längsrichtung des Bedienungsgriffs 10 parallelen Breitseitenflächen paarweise an entgegengesetzten Stirnenden des Tragelements 20 und auf entgegengesetzten Seiten des Tragelements 20 angeordnet, wobei ihre aktiven Flächen 28 jeweils in Kompressionsrichtung zu einem der Kraftübertragungskörper 26 hin gerichtet sind. Die Foliendrucksensoren 8a,8b,8c,8d sind fest mit dem Tragelement 20 einerseits und mit dem zugehörigen Kraftübertragungskörper 26 andererseits verbunden, wobei die Verbindung beispielsweise durch Aufkleben der Foliendrucksensoren 8a,8b,8c,8d auf das Tragelement 20 und anschließendes Aufkleben der Kraftübertragungskörper 26 auf die Foliendrucksensoren 8a,8b,8c,8d oder durch Einbetten der auf das Tragelement aufgeklebten Foliendrucksensoren 8a,8b,8c,8d im Kraftübertragungskörper 26 hergestellt werden kann.

Das Griffteil 24 ist unter geringfügiger Verformung von jeweils zwei der Kraftübertragungskörper 26 sowohl in Längsrichtung des Tragelements 20 (entsprechend der Längsrichtung des Patientenlagerungstisches 2) als auch quer dazu (entsprechend der Kompressionsrichtung) gegenüber dem Tragelement 20 begrenzt hin- und herverschiebbar und liegt mit zwei als Kraftangriffsflächen dienenden Kraftangriffsflächen 30,32 an jedem der Kraftübertragungskörper 26 an. Während die an gegenüberliegenden Stirnflächen 34 der Kraftübertragungskörper 26 anliegenden Kraftangriffsflächen 30 senkrecht zur Längsrichtung des Bedienungsgriffs 10 und damit zur Längsrichtung des Patientenlagerungstisches 2 ausgerichtet sind, weisen die gegen eine halbzylindrischen Umfangsfläche 38 der Kraftübertragungskörper 26 anliegenden Kraftangriffsflächen 32 eine der Form der Umfangsfläche 38 entsprechende halbzylindrische Form auf.

Die aus einem weichen elastomeren Werkstoff hergestellten Kraftübertragungskörper 26 sind so im Bedienungsgriff 10 integriert, daß sie bei Beaufschlagung des Bedienungsgriffs 10 mit einer Kraft in Längsrichtung des Patientenlagerungstisches 2 oder in Kompressionsrichtung keine Ausweichmöglichkeiten besitzen, d.h. sie werden allseitig von den Kraftangriffsflächen 30,32, von einer oder mehreren, den Kraftangriffsflächen 30,32 gegenüberliegenden Widerlagerflächen des Tragelements 20, sowie vom Foliendrucksensor 8a,8b,8c,8d begrenzt. Die Kraftübertragungskörper 26 verhalten sich daher wie eine inkompressible Flüssigkeit, d.h. eine auf ihre Begrenzungsfläche 34,38 einwirkende Kraft wird entsprechend dem Verhältnis aus der Kraft und dem zur Kraftrichtung senkrechten Flächenanteil in einen Druck im Kraftübertragungskörper 26 umgewandelt, der sich allseitig im gesamten Kraftübertragungskörper 26 fortpflanzt und an seinen sämtlichen Begrenzungsflächen wirkt, d.h. auch an einer Begrenzungsfläche 40 des Kraftübertragungskörpers 26, die an der aktiven Fläche 28 des Foliendrucksensors 8a,8b,8c,8d anliegt.

Da diese Begrenzungsfläche 40 sowohl bei Kräften in Längsrichtung des Bedienungsgriffs 10 (F_{längs}) als auch bei Kräften in Kompressionsrichtung (Fₖₒₘₚ) zur Übertragung des Druck auf den Foliendrucksensor 8a,8b,8c,8d dient, ist die Verstärkung der Kräfte ausschließlich von der Größe der Kraftangriffsflächen 30,32 bzw. der Größe von deren Projektionen in einer zur Richtung von F_{längs} bzw. Fₖₒₘₚ senkrechten Richtung abhängig. Wenn also die Kraftangriffsflächen 30 und 32 unterschiedlich groß sind, führt eine in Längsrichtung bzw. in Kompressionsrichtung auf das Griffteil 24 ausgeübte gleichgroße Kraft zu einem unterschiedlich großen Druck in den jeweils mit Kraft beaufschlagten Kraftübertragungskörpern 26 und damit zu einem unterschiedlich großen Spannungssignal im Foliendrucksensor 8, was wiederum eine unterschiedlich große Kraftverstärkung durch den Servomotor zur Folge hat.

Durch entsprechende Bemessung der Kraftangriffsflächen 30,32 bzw. ihrer Projektionen in einer zur jeweiligen Kraftkomponente in den Bewegungsrichtungen des Röntgenzielgerätes 4 senkrechten Richtung können somit unterschiedliche Empfindlichkeiten der Anordnung kompensiert oder korrigiert werden, die beispielsweise dadurch entstehen, daß die in Längsrichtung des Patientenlagerungstisches 2 bzw. in Kompressionsrichtung zu verschiebenden Trägheitsmassen des Röntgenzielgerätes 4 unterschiedlich groß sind. Eine derartige Korrektur weist gegenüber einer rechnerischen Korrektur oder einer unterschiedlichen Verstärkung der Signale zu Korrekturzwecken den Vorteil einer größeren Robustheit, einer geringeren Störanfälligkeit und einer einfacheren Justierung auf.

Zur Erläuterung entspricht bei dem in Figur 2 und 3 dargestellten Ausführungsbeispiel die Angriffsfläche 30 für F_{längs} jeweils ungefähr der an der aktiven Fläche 28 des Foliendrucksensors 8a,8b,8c,8d anliegenden Begrenzungsfläche 40 des Kraftübertragungskörpers 26, während die Angriffsflächen 32 für Fₖₒₘₚ jeweils erheblich größer sind, so daß bei Übereinstimmung des Betrags von F_{längs} und Fₖₒₘₚ die druckinduzierte Widerstandabnahme durch F_{längs} erheblich höher ist. Neben einer unterschiedlichen Trägheitsmasse lassen sich damit beispielsweise auch die einseitige Anordnung des Bedienungsgriffs 10 am Röntgenzielgerät 4 und das dadurch bedingte Drehmoment kompensieren. Durch eine senkrecht zu F_{längs} und Fₖₒₘₚ verschobene Anordnung der Foliendrucksensoren 8a,8b,8c,8d und der Kraftübertragungskörper 26 im Bedienungsgriff 10, und eine einseitige Einspannung des Tragelements 20 im Griffteil 24, wie in Figur 4 dargestellt, kann dem ebenfalls Rechnung getragen werden.

Durch eine geeignete Verstärkung von F_{längs} und Fₖₒₘₚ wird dem Arzt bei einem Verschieben des Röntgenzielgerätes 4 in einer beliebigen der vier möglichen Bewegungsrichtungen das Gefühl vermittelt, nur eine sehr kleine Trägheitsmasse zu bewegen, wobei aufgrund der im wesentlichen proportionale Verstärkung infolge der in Figur 7 dargestellten annähernd linearen Kennlinie der Foliendrucksensoren 8a,8b,8c,8d natürlichen Bewegungsmustern Rechnung getragen wird, indem große Kräfte entsprechend stärker verstärkt werden als kleine Kräfte.

In einer zur Längsrichtung des Patientenlagerungstisches 2 und zur Kompressionsrichtung senkrechten Richtung ist das Griffteil 24 gegenüber dem Tragelement 20 unverschiebbar gelagert, d.h. es liegt unmittelbar ohne Zwischenschalten der Kraftübertragungskörper 26 am Tragelement 20 an, so daß in dieser Richtung in das Griffteil 24 eingeleitete Kräfte nicht zu einer Druckerhöhung in einem der Kraftübertragungskörper 26 und damit nicht zu einem Ansprechen der Servomotoren 5,7 führen.

Bei Beaufschlagung des Bedienungsgriffs 10 mit einer Kraft in einer der beiden entgegengesetzten Richtungen von F_{längs} bzw. von Fₖₒₘₚ wird diese Kraft jeweils immer in zwei Kraftübertragungskörper 26 eingeleitet. Bei einer Kraft F_{längs} in Figur 2 nach links werden beispielsweise die beiden Kraftübertragungskörper 26a und 26b mit Druck belastet und damit der Widerstand der beiden Foliendrucksensoren 8a und 8b verändert, während bei einer Kraft F_{längs} in Figur 2 nach rechts die beiden Kraftübertragungskörper 26c und 26d mit Druck beaufschlagt und der Widerstand der Foliendrucksensoren 8c und 8d verändert wird. Entsprechend werden bei einer Kraft Fₖₒₘₚ in Figur 2 nach unten die beiden Kraftübertragungskörper 26a und 26c mit Druck belastet und der Widerstand der Foliendrucksensoren 8a und 8c verändert, während durch eine Kraft Fₖₒₘₚ in Figur 2 nach oben die beiden Kraftübertragungskörper 26b und 26d mit Druck beaufschlagt und der Widerstand der beiden Foliendrucksensoren 8b und 8d verändert wird. Die Kraftangriffsflächen 30,30 bzw. 32,32 der jeweils gemeinsam beaufschlagten Kraftübertragungskörper 26a,26b; 26c,26d bzw. 26a,26c; 26b,26d sind jeweils gleich groß, so daß die Druckerhöhung im Kraftübertragungskörper 26a,26b; 26c,26d; 26a,26c bzw. 26b,26d und die damit verbundenen Widerstandsabnahme in den jeweils zugehörigen beiden Foliendrucksensoren 8a,8b; 8c,8d; 8a,8c bzw. 8b,8d ebenfalls gleich groß ist.

Eine zur Umsetzung der Widerstandsänderungen in Steuersignale für die Servomotoren geeignete Schaltung ist in Fig. 7 skizziert. Danach sind die Sensoren 8a ... 8d Bestandteil je eines an eine Gleichspannung angeschlossenen Spannungsteilers. Die Spannungen an den Abgriffen, die umso größer sind, je größer der Druck auf den zugehörigen Sensor ist, werden über einen Multiplexer 9 und einen A/D-Wandler 11 einem Mikrocontroller 12 zugeführt. Dieser steuert über Regler 50 und 70 die Servomotoren 50 und 70 hinsichtlich Drehzahl und Drehrichtung.

Der Mikrocontroller 12 ist so programmiert, daß ermittelt wird, ob zwei annähernd gleiche, von Null verschiedene Spannungen vorliegen und zu welchen Sensoren diese Spannungen gehören. Liegen beispielsweise zwei Spannungen von den Sensoren 8c und 8c vor, wird über den Regler 70 der Servomotor 7 so aktiviert, daß eine Verschiebung des Zielgerätes in Längsrichtung nach rechts erfolgt; stammen die Spannungen von zwei anderen Sensoren, erfolgt eine Verschiebung in einer anderen Richtung. Die Drehzahl hängt dabei von der Größe der Spannungen ab.

Werden zwei stark voneinander abweichende Spannungen oder nur eine eine Schwelle überschreitende Spannung ermittelt, ist dies ein Hinweis auf einen Defekt oder einen Ausfall eines Sensors. Durch Abschalten der Servomotoren 5a,5b,7 kann in einem derartigen Fall automatisch verhindert werden, daß ein Patient oder Arzt durch eine ungewollte Bewegung des Röntgenzielgerätes verletzt wird.

Der in Figur 5 dargestellte Bedienungsgriff 10 kann an einem Röntgenzielgerät 4 eingesetzt werden, das in Richtung von F_{längs} und von Fₖₒₘₚ sowie in einer dazu senkrechten Richtung verschiebbar ist. Durch eine asymmetrische Ausbildung des Tragelements 20 und der Kraftübertragungskörper 26 wird dort an jedem Kraftübertragungskörper 26 eine weitere Kraftangriffsfläche 50 gebildet, die einer Widerlagerfläche 52 des Tragelements 20 gegenüberliegt, so daß bei Beaufschlagung des Griffteils 24 mit einer zu F_{längs} und zu Fₖₒₘₚ senkrechten Kraft F_{quer} in jeweils zwei der Kraftübertragungskörper 26 ein Druck erzeugt wird, der eine Widerstandsänderung des Foliendrucksensors 8a,8b,8c,8d zur Folge hat.

Entsprechend lassen sich das Tragelement 20, das Griffteil 24, und die Kraftübertragungskörper 26 auch derart ausbilden und zusammen mit vier Foliendrucksensoren 8a,8b,8c,8d im Bedienungsgriff 10 anordnen, daß an Stelle einer Bewegung senkrecht zu F_{längs} und zu Fₖₒₘₚ eine Schwenkbewegung um eine zu F_{längs} und Fₖₒₘₚ senkrechte Drehachse und eine entsprechende Kraftverstärkung in Drehrichtung mittels eines weiteren Servomotors möglich ist.

## Patentansprüche

1. Röntgenuntersuchungsgerät mit einem Patientenlagerungstisch und einem relativ zum Patientenlagerungstisch in mindestens einer Raumrichtung hin- und herbeweglichen Röntgenzielgerät, sowie durch einen Bedienungsgriff am Röntgenzielgerät aktivierbaren Sensoren zum Ansteuern mindestens eines Servomotors des Röntgenzielgerätes,
dadurch gekennzeichnet, daß das Röntgenzielgerät mit dem Bedienungsgriff in mindestens zwei unterschiedlichen Raumrichtungen verschiebbar ist, und daß die Sensoren (8a,8b,8c,8d) in Bezug auf den Bedienungsgriff (10) so angeordnet sind, daß bei Beaufschlagung des Bedienungsgriffs (10) mit einer Kraft in einer beliebigen der mindestens vier möglichen Bewegungsrichtungen jeweils eine andere Gruppierung (8a,8b; 8c,8d; 8a,8c; 8b,8d) aus jeweils mindestens zwei der Sensoren (8a,8b,8c,8d) aktiviert wird.

2. Röntgenuntersuchungsgerät nach Anspruch 1,
dadurch gekennzeichnet, daß das Röntgenzielgerät (4) zusätzlich in einer von den beiden Raumrichtungen verschiedenen dritten Raumrichtung hin- und herbeweglich ist, wobei die Sensoren (8a,8b,8c,8d) so angeordnet sind, daß bei Beaufschlagung des Bedienungsgriffs (10) mit einer Kraft in einer beliebigen der sechs möglichen Bewegungsrichtungen jeweils eine andere Gruppierung (8a,8b; 8c,8d; 8a,8c; 8b,8d; 8a,8d; 8b,8c) aus jeweils mindestens zwei Sensoren (8a,8b,8c,8d) aktiviert wird.

3. Röntgenuntersuchungsgerät nach Anspruch 1,
dadurch gekennzeichnet, daß das Röntgenzielgerät (4) zusätzlich um eine Drehachse verschwenkbar ist, wobei die Sensoren (8a,8b,8c,8d) so angeordnet sind, daß bei Beaufschlagung des Bedienungsgriffs (10) mit einer Kraft in einer beliebigen der vier möglichen linearen Bewegungsrichtungen oder in einer beliebigen der beiden möglichen Drehrichtungen jeweils eine andere Gruppierung (8a,8b; 8c,8d; 8a,8c; 8b,8d; 8a,8d; 8b,8c) aus jeweils mindestens zwei Sensoren (8a,8b,8c,8d) aktiviert wird.

4. Röntgenuntersuchungsgerät nach einem der Ansprüche 1 bis 3,
dadurch gekennzeichnet, daß die einzelnen Sensor-Signale einer Auswerteschaltung zugeführt werden, die den Servomotoren (5,7) nur dann Antriebsenergie zuführt, wenn alle Sensorsignale jeweils einer Gruppierung (8a,8b; 8c,8d; 8a,8c; 8b,8d bzw. 8a,8b; 8c,8d; 8a,8c; 8b,8d; 8a,8d; 8b,8c) zumindest annähernd vergleichbar groß sind.

5. Röntgenuntersuchungsgerät nach Anspruch 1,
gekennzeichnet durch mindestens einen zwischen dem Bedienungsgriff (10) und jedem Sensor (8a,8b,8c,8d) angeordneten, verformbaren Kraftübertragungskörper (26a,26b,26c,26d) aus einem inkompressiblen Material mit vorzugsweise elastischen Eigenschaften.

6. Röntgenuntersuchungsgerät nach Anspruch 5,
dadurch gekennzeichnet, daß die Sensoren (8a,8b,8c,8d) und die zugeordneten Kraftübertragungskörper (26a,26b,26c,26d) zwischen einem starr mit dem Röntgenzielgerät (4) verbundenen Tragelement (20) und einem mit einer Griffoberfläche (22) versehenen Griffteil (24) des Bedienungsgriffs (10) angeordnet sind.

7. Röntgenuntersuchungsgerät nach Anspruch 6,
dadurch gekennzeichnet, daß die Kraftübertragungskörper (26a,26b,26c,26d) zu den möglichen Bewegungsrichtungen vorzugsweise senkrechte Kraftangriffsflächen (30,32,50) für das Griffteil (24) aufweisen.

8. Röntgenuntersuchungsgerät nach Anspruch 6,
dadurch gekennzeichnet, daß eine Beaufschlagung des Bedienungsgriffs (10) mit einer Kraft oder Kraftkomponente in einer zu den möglichen Bewegungsrichtungen des Röntgenzielgerätes (4) senkrechten Richtung nicht zu einer Druckerhöhung in einem der Kraftübertragungskörper (26a,26b,26c,26d) führt.

9. Röntgenuntersuchungsgerät nach Anspruch 6,
dadurch gekennzeichnet, daß die Sensoren (8a,8b,8c,8d) jeweils paarweise an entgegengesetzten Enden des Bedienungsgriffs (10) und auf entgegengesetzten Seiten des Tragelements (20) angeordnet sind.

10. Röntgenuntersuchungsgerät nach Anspruch 1,
dadurch gekennzeichnet, daß die Sensoren (8a,8b,8c,8d) als Foliendrucksensor ausgebildet sind.
